# EUROPEAN PATENT SPECIFICATION

(11) **EP 1 377 604 B1**
(45) Date of publication and mention of the grant of the patent: **31.12.2014**
(21) Application number: 02722972.3
(22) Date of filing: 11.04.2002
(51) Int. Cl.: C12N 5/07

(54) **METHODS AND MEANS FOR USE OF HLA-DQ RESTRICTED T-CELL RECEPTORS AND HLA-DQ-BINDING PROLAMINE-DERIVED PEPTIDES**
METHODEN UND MITTEL ZUR VERWENDUNG VON AUF HLA-DQ BESCHRÄNKTEN T-ZELL REZEPTORENSOWIE HLA-DQ-BINDENDEN, VON PROLAMIN ABSTAMMENDEN, PEPTIDEN
PROCEDES ET MOYENS A UTILISER POUR DES RECEPTEURS DE CELLULES T RESTREINTES AU HLA-DQ ET PEPTIDES DERIVES DE PROLAMINE SE LIANT A HLA-DQ

(30) Priority: 12.04.2001 EP 01201377; 16.11.2001 EP 01204383
(43) Date of publication of application: 07.01.2004
(73) Proprietor: Academisch Ziekenhuis Leiden, 2333 ZA Leiden (NL)
(72) Inventor: DRIJFHOUT, Jan, Wouter, NL-2321 AK Leiden (NL); KONING, Frits, NL-2353 WR Leiderdorp (NL); McADAM, Stephan, Neil, NL-1341 Slependen (NO); SOLLID, Ludvig, Magne, NL-1357 Bekkestua (NO)
(74) Representative: Prins, Adrianus Willem
(86) International application number: PCT/NL2002/000235
(87) International publication number: WO 2002/083722

(56) References cited:
- EP-A- 0 905 518
- JOHANSEN B H ET AL: "BINDING OF PEPTIDES FROM THE N-TERMINAL REGION OF ALPHA-GLIADIN TO THE CELIAC DISEASE-ASSOCIATED HLA-DQ2 MOLECULE ASSESSED IN BIOCHEMICAL AND T CELL ASSAYS" CLINICAL IMMUNOLOGY AND IMMUNOPATHOLOGY, SAN DIEGO, CA, US, vol. 79, no. 3, 1 June 1996 (1996-06-01), pages 288-293, XP002072057 ISSN: 0090-1229
- GJERTSEN HENRIK A ET AL: "T cells recognize a peptide derived from alpha-gliadin presented by the celiac disease: Associated HLA-DQ (alpha-*-0501,beta-1*0201) heterodimer." HUMAN IMMUNOLOGY, vol. 39, no. 4, 1994, pages 243-252, XP001025863 ISSN: 0198-8859
- WAL VAN DE Y ET AL: "SMALL INTESTINAL T CELLS OF CELIAC DISEASE PATIENTS RECOGNIZE A NATURAL PEPSIN FRAGMENT OF GLIADIN" PROCEEDINGS OF THE NATIONAL ACADEMY OF SCIENCES OF USA, NATIONAL ACADEMY OF SCIENCE. WASHINGTON, US, vol. 95, no. 17, August 1998 (1998-08), pages 10050-10054, XP000982626 ISSN: 0027-8424
- ARENTZ-HANSEN H ET AL: "THE INTESTINAL T CELL RESPONSE TO ALPHA-GLIADIN IN ADULT CELIAC DISEASE IS FOCUSED ON A SINGLE DEAMIDATED GLUTAMINE TARGETED BY TISSUE TRANSGLUTAMINASE" JOURNAL OF EXPERIMENTAL MEDICINE, TOKYO, JP, vol. 191, no. 4, 21 February 2000 (2000-02-21), pages 603-612, XP000986723 ISSN: 0022-1007
- ANDERSON R P ET AL: "IN VIVO ANTIGEN CHALLENGE IN CELIAC DISEASE IDENTIFIES A SINGLE TRANSGLUTAMINASE-MODIFIED PEPTIDE AS THE DOMINANT A-GLIADIN T-CELL EPITOPE" NATURE MEDICINE, NATURE AMERICA, NEW YORK, US, vol. 6, no. 3, March 2000 (2000-03), pages 337-342, XP000982628 ISSN: 1078-8956
- VADER L W ET AL: "The gluten-specific T cell response in pediatric celiac disease patients." IMMUNOBIOLOGY, vol. 203, no. 1-2, November 2000 (2000-11), page 521 XP001021312 Joint Annual Meeting of the German and Dutch Societies of Immunology;Duseldorf, Germany; November 29-December 02, 2000 ISSN: 0171-2985
- MCADAM S N ET AL: "Getting to grips with gluten", GUT, vol. 47, no. 6, December 2000 (2000-12), pages 743-745, ISSN: 0017-5749
- PARTANEN J: "The HLA-DRB4 gene does not explain genetic susceptibility in HLA-DQ2-negative celiac disease.", IMMUNOGENETICS MAR 2000, vol. 51, no. 3, March 2000 (2000-03), pages 249-251, ISSN: 0093-7711
- GARROTE JOSE A ET AL: "The HLA-DRB4 gene is present in half of the Spanish HLA-DQ2-negative celiac patients", IMMUNOGENETICS, vol. 51, no. 12, October 2000 (2000-10), pages 1045-1046, ISSN: 0093-7711
- KARELL KATI ET AL: "HLA types in celiac disease patients not carrying the DQA1*05-DQB1*02 (DQ2) heterodimer: results from the European Genetics Cluster on Celiac Disease", 20030401; 20030400, vol. 64, no. 4, 1 April 2003 (2003-04-01), pages 469-477, XP002456767,

## Description

The invention relates to the field of molecular biology and immunology. More specific the invention relates to food-related immune enteropathies such as celiac sprue, tropical sprue, giardiasis and food allergies of childhood.

As one of the main representatives of this family of diseases, we will describe celiac disease (CD) or celiac sprue in greater detail as representative of the applications of the present invention. Celiac disease (CD) or celiac sprue is a disorder of the small intestine characterised by crypt-cell hyperplasia and villous atrophy, accompanied by an increased number of intraepithelial lymphocytes. Characteristic symptoms are a mild to severe malabsorption syndrome, diarrhea, cachexia and weight loss but can sometimes include lymphoma or other types of cancer. The disease is caused by a sensitivity to gluten (prolamine) and is precipitated in susceptible individuals by ingestion of cereal proteins.

Current therapy of CD mainly involves dietary treatment of gluten-sensitive patients with diets lacking cereal compounds such as flour, which deprives these patients of such typical staple foods as for example bread. Wheat gluten comprises a mixture of two proteins, glutenins and gliadins, which contain 35-45% glutamine (Q) and 12-20% proline (P). Glutenins are of high molecular weight, comprising approximately 500-1000 amino acids, covalently bound head-to-tail by disulfide bridges, forming multimeric complexes. Glutenins are responsible for the elasticity and extensibility of the gluten. The gliadins are of lower molecular weight, comprising approximately 250-600 amino acids, are monomeric, and are responsible for the viscosity of the gluten.

The criteria for glutenfree products are established by the Codex Alimentarius Committee "Nutrition and Food for Special Dietary Uses" that meets every 1.5 year. The current criterion is based on the determination of nitrogen. To be considered glutenfree a product may contain maximally 50 mg N per 100 gram of product. The determination of nitrogen is only useful when there is a certain relationship between the amount of nitrogen and the amount of gluten. This is only true, to a certain extent, for wheat.

By determining the amount of gluten (by nitrogen measurement) no relevant data are obtained on the amount of toxic compound which is actually involved in the development and persistency of the food related immune enteropathy. The present invention recognizes this problem and discloses means and methods to determine the amount of (toxic) prolamine-derived peptides involved in food related immune enteropathy. The present invention also discloses novel pharmaceuticals based on the identified prolamine-derived peptides.

The invention provides an isolated, recombinant or synthetic peptide comprising the amino acid sequence PFRPEQPYPQPQPQ, optionally coupled to a carrier molecule, wherein said prolamine-derived peptide is involved in food-related immune enteropathy. Such a peptide is preferably capable of associating with an HLA-DQ molecule (or more preferably with an HLA-DQ2 or HLA-DQ8 molecule), thereby facilitating recognition by an isolated or recombinant HLA-DQ restricted T-cell receptor according to the invention. Carrier is herein defined as a component providing a prolamine-derived peptide with the capacity of inducing a proper immune response. Examples of such a carrier are keyhole limpet hemocyanin (KLH) or human serum albumine (HSA). The person skilled in the art is aware of the fact that there exist many more carrier molecules. Production of synthetic and/or recombinant peptides is well known within the art. Examples for the production of synthetic peptides and methods to isolate prolamine-derived peptides from for example gluten are disclosed herein within the experimental part. Preferred is an isolated, an isolated, recombinant or synthetic prolamine-derived peptide or a functional equivalent and/or a functional fragment thereof, optionally coupled to a carrier molecule, wherein said prolamine-derived peptide is involved in food-related immune enteropathy and wherein said prolamine-derived peptide is obtainable from a protein selected from the group of gliadins, glutenins, secalins, hordeins or avenins. Now that these specific peptides are disclosed, it is easy to determine the corresponding processing sites which are used by for example proteases. Knowledge of these processing sites is used to construct proteins (for example via recombinant DNA technology) which are no longer processed, thereby inhibiting the production of prolamine-derived peptides involved in food-related immune enteropathy. In another embodiment the invention provides an isolated, recombinant or synthetic prolamine-derived peptide according to the invention, wherein said prolamine-derived peptide is flanked by amino acids representing antigen-processing sites. Said food-related immune enteropathy is preferably selected from the group of celiac sprue, tropical sprue, giardiasis or food allergies of childhood.

The invention also provides an isolated or synthetic antibody specifically recognising the amino acid sequence PFRPEQPYPQPQPQ in a prolamine-derived peptide according to the invention. Such a peptide is preferably capable of associating with an HLA-DQ molecule, thereby facilitating recognition by an isolated or recombinant HLA-DQ restricted T-cell receptor according to the invention. Such an antibody is for example obtainable by immunising an immuno-competent animal with a prolamine-derived peptide according to the invention and harvesting polyclonal antibodies from said immunised animal, or obtainable by other methods known in the art such as by producing monoclonal antibodies, or (single chain) antibodies or binding proteins expressed from recombinant nucleic acid derived from a nucleic acid library, for example obtainable via phage display techniques.

With such an antibody, the invention also provides an immunoassay comprising an antibody according to the invention. A lot of immunoassays are available within the art, for example ELISA (Enzyme Linked Immuno Sorbent Assay) or Western blotting.

In yet another embodiment the invention provides a diagnostic kit comprising an antibody according to the invention and a suitable means of detection. Such a diagnostic kit is, for example, very useful for detecting in food, food components or samples from (suspected) patients the presence of a prolamine-derived peptide involved in food-related immune enteropathy (for example: celiac sprue, tropical sprue, giardiasis or food allergies of childhood). At present such a quantitative and qualitative diagnostic kit determining the presence and/or amount of prolamine-derived peptide is not available. Currently two different assays are used for gluten detection. One assay determines the nitrogen content of food (components) as a measure for the presence of gluten. The other assay utilises gluten specific antibodies in ELISA systems. However, both assay systems do not test for the toxic prolamine-derived peptides involved in food-related immune enteropathy. A diagnostic kit comprises, for example, an antibody according to the invention specifically recognising a toxic prolamine-derived peptide involved in food-related immune enteropathy. Another advantage of the diagnostic kit as described in the present application is the capability of testing food (components) which cannot be tested or cannot be tested reliably by the currently used gluten assays. The existing assays are hardly informative when food (components) contain significant amounts of other nitrogen containing compounds (e.g. other proteins) or when food (components) contain partially hydrolysed prolamine proteins that are not recognised by antibodies currently used in ELISA-kits. Examples of food (components) for which the existing assays are troublesome are beer, melassis and soy sauce. In addition, the existing assays lack the level of sensitivity required for many applications.

Preferably a diagnostic kit uses different antibodies, each capable of recognizing another prolamine-derived peptide involved in food-related immune enteropathy. Thereby multiple prolamine-derived peptides involved in food-related immune enteropathy are detected. In the art different kinds of means of detection are available and the skilled person knows how to select a proper means of detection. Examples are chromogenic or fluorigenic substances. The invention thus provides methods and means for the monitoring of a T-cell reactive component in food, food component or samples from (suspected) patients.

The invention further provides a method to decrease the amount of toxic prolamine-derived peptides in food or food components comprising incubating an antibody according to the invention with said food or food component. For example an antibody according to the invention is coupled to appropriate carrier material (for example free beads or column material) and the food or food component is brought in contact with the coupled antibody. The amount of prolamine-derived peptides involved in food related immune enteropathy is reduced (preferably completely diminished) to an acceptable level. Preferably a method is used to decrease the amount of prolamine-derived peptides which are obtainable from proteins like gliadines, glutenins, secalins, hordeins or avenins.

Furthermore the invention provides a method to select a cereal comprising providing an antibody according to the invention, whereby a cereal lacking at least one prolamine-derived peptide according to the invention is selected. Such a method to select a cereal comprises for example the next steps. Gluten, isolated from a particular wheat strain, is digested with an appropriate enzyme or with a mixture of enzymes. An antibody according to the invention is used in an immunoassay to detect toxic prolamine-derived peptides in said digested gluten preparation. By comparing multiple wheat strains/variaties for the presence/absence of prolamine-derived peptides (involved in food-related immune entheropathy), wheat strains are selected which are useful for breeding experiments. Cereals are selected for the presence or absence of prolamine-derived peptides. Such selected cereals are than produced via agricultural and/or industrial methods into food or food components for gluten sensitive individuals. Cereal, in this application, relates to grain or related grasses or plants that produce it and to the (prepared) foodstuff. In particular wheat gluten, but also rye, and to a lesser extent barley and oat may cause disease. Because prolamine-derived peptides involved in food-related immune enteropathy are described herein, one is now able to genetically modify the genome of cereals to generate new cereals with a decreased source of toxic prolamine-derived peptide. Modifications are, for example, generated by point-mutations in the nucleic acid sequence of the prolamine or are generated by replacing such a sequence by another sequence not giving rise to prolamine-derived peptides involved in food-related immune enteropathy. A cereal selected and/or bred according to a method of the invention is used to prepare food low or preferably free of prolamine-derived peptides involved in food-related immune enteropahty.

In another embodiment the invention provides a pharmaceutical composition comprising a prolamine-derived peptide according to the present invention. Such a pharmaceutical composition is used for the induction of tolerance against said prolamine-derived peptide. For tolerance induction doses of a prolamine-derived peptide according to the invention are given repeatedly, for instance intravenously, but other routes of administration are suitable too. Another possibility is the repeated oral or nasal administration of such a prolamine-derived peptide. Such a prolamine-derived peptide according to the present invention is given alone, or in combination with other (toxic) prolamine-derived peptides, or as part of larger molecules, or coupled to carrier materials/molecules. A pharmaceutical composition comprising a prolamine-derived peptide according to the present invention is also used for elimination of a certain subset of T-cells or for the treatment of gluten-sensitivity. Preferably such a pharmaceutical composition according to the present invention contains various, different kinds of, prolamine-derived peptides.

### EXPERIMENTAL PART

### Children with CD.

Twenty-two caucasiod CD patients were included in the present study. Their age at diagnosis (first small bowel biopsy) was between 1 and 9 years old (average age 3.6 years, SD 1.8; 1 year old, 3 patients; 2 years old, 3 patients; 3 years old, 8 patients; 4 years old, 5 patients; 6 years old, 2 patients; 9 years old, 1 patient). All the patients expressed the disease associated DQ2 allele encoded by DQA1*05/DQB1*02.

### Antigens and peptides.

A pepsin/trypsin digest of gluten was prepared as described [1]. Peptides were synthesised by standard Fmoc chemistry on a multiple peptide synthesiser (SyroII). Integrity of synthetic peptides was checked by rpHPLC and mass spectrometry. Tissue transglutaminase (tTG) treatment was performed by incubating the peptides with this enzyme (Sigma; T-5398) at a concentration of 500 µg/ml and 100 µg/ml respectively at 37°C for 4 h minimum, in 50 mM TEA-acetate pH 6.5, 2 mM CaCl₂.

### Isolation of gluten specific T cell lines.

Polyclonal gluten specific T cell lines were generated from small intestinal biopsy of the celiac disease patients as described [1, 2]. In short, small intestinal biopsies were cultures with either the trypsin/pepsin treated gluten preparation or a tTG/trypsin/pepsin treated gluten preparation. After one round of restimulation with the gluten preparations in the presence of autologous PBMC the cells were expanded with IL-2, tested for specificity and frozen until further use. T cell clones were generated as described previously [1, 2]. In proliferation assays in which matched and mismatched APC were used it was found that the T cell lines and/or clones responded to stimulation with gluten preparation in the presence of HLA-DQ2 positive APC only. Moreover, the response could be blocked with DQ-specific antibodies. The parents of all patients gave informed consent to the study, which was approved by the hospital ethics committee.

### T cell proliferation assays.

Proliferation assays were performed in duplicate in 150 µl culture medium (RPMI1640 [Gibco], containing 10% human serum) in 96-well flat-bottomed plates (Falcon) using 10⁴ T cells stimulated with 10⁵ irradiated PBMCs (3000 RAD) in the presence or absence of antigen at the indicated concentrations. After 48 hours, cultures were pulsed with 0.5 µCi of ³H-thymidine and harvested 18 hours thereafter. Another way to perform a T cell proliferation assay is described below.

Triplicate wells with irradiated APC were incubated overnight with antigen in U-bottomed 96 well plates in a total volume of 100 µl before T cells (5 x 10⁴) in a volume of 50 µl were added. [³H]-thymidine was added 2 days later and the plates were incubated further 12-16 h before [³H]-thymidine incorporation was counted on a Betaplate Counter (Wallac Turku). DR3⁺DQ2⁺ B lymphoblastoid cells (irradiated 80 Gy) was used as APC. HLA restriction of the TCC was first determined by comparing the proliferative response to a peptide pulsed, DQA1*05/DQB1*0301 positive B-LCL SWEIG with and without an additional transfected DQB1*0201 chain. This restriction was confirmed by inhibition of T cell activation with a monoclonal antibody (SPV-L3, DQ monomorphic) using a DQA1*05/DQB1*0201 homozygous B-LCL as APC.

Results obtained from both T cell proliferation assay provide comparable data.

### HPLC purification of the pepsin/trypsin digest of gluten.

Approximately 1 mg of an enzymatic digest of gluten was fractionated via micro-rpHPLC (SMART system, column C2/C18, sc 2.1/10, Pharmacia) using an acetonitrile gradient from 0 to 70% (2%/min, flow rate 100 µl/min, containing 0.1% trifluoroacetic acid). The second dimension of fractionation by rpHPLC was performed with a gradient of 0.5% acetonitrile per min, and in a third round trifluoroacetic acid was replaced with 0.1% heptafluorobutyric acid.

### Mass spectrometry.

Electrospray ionization mass spectrometry was performed on the most abundant peaks present in the bioactive HPLC fraction using a Q-TOF hybrid mass spectrometer (Micromass, Manchester, UK) as described [1, 2]. Briefly, precursors were selected with the quadrupole and fragments were collected with high efficiency with the orthogonal time of flight mass spectrometer. The collision gas applied was argon (pressure 4x10⁻⁵ mbar) and the collision voltage approximately 30 V. Another way to perform mass spectrometry is described below.

Electrospray ionization (ESI) mass spectra were recorded on a quadrupole-Time-of-Flight (Q-TOF) mass spectrometer (Micromass, Manchester, UK) and ion matrix-assisted laser desorption ionisation (MALDI) spectra were acquired on a Bruker Reflex II MALDI-TOF instrument (Bruker-Daltonik, Bremen, Germany). After purification, the samples were sprayed from nanoelectrospray needles (MDS Proteomics, Odense, DK) held at 800 V towards a skimmer cone (40 V). In collision-induced dissociation (CID) experiments (8.7 x 10⁻⁵ mBar argon, collision energy 32 to 40 eV), product ions were analyzed by the orthogonal TOF analyzer.

Results obtained from both methods provide comparable data.

### Database searching.

The program PeptideSearch was used for sequence elucidation. Database similarity searches were done on the basis of the newly identified gluten peptide sequences by FASTA searches in a selected subset of wheat proteins from the Swiss Prot databank.

### Adult coeliac patients.

Thirteen adult celiac disease patients were included in the study, which was approved by the regional ethical committee. Patient CD411 and CD410 were untreated, whereas patients CD380, CD377, CD421, CD370, CD387, CD423, CD429, CD430, CD432, CD436 and CD450 were on gluten free diet. All subjects expressed the disease associated DQ2 molecule encoded by DQA1*05/DQB1*02 alleles.

### Amplification, cloning and production of recombinant γ-gliadins.

The amplification, cloning and production of recombinant gliadins was performed as previously described [6]. Briefly, amplification from genomic DNA isolated from the Nordic wheat strain Mjølner was performed using primers designed to amplify full-length mature γ-gliadin. PCR products of appropriate size were cloned into the pET17xb expression vector. Cycle sequencing of gliadin clones were performed on PCR products using the Thermo Sequenase dye terminator cycle sequencing pre-mix kit (Amersham Pharmacia Biotech) according to the manufacturers manual. Sequencing products were run on an ABI Prism 377XL DNA sequencer (Perkin Elmer, Norwalk, Connecticut, USA). Plasmids containing full-length γ-gliadin genes were expressed in *E. coli* using the pET expression system. Gliadin was extracted from *E. coli* by incubation in 70% ethanol at 60°C for 2 hours and precipitated by addition of NaCl to a final concentration of 1 M. Analysis of the gliadin preparations on Coomassie Blue stained SDS PAGES revealed dominant bands of the appropriate weight with only minor contaminations.

### Biochemical purification of fragments from recombinant gliadin stimulatory for T cells.

The method for preparation of T cell active gliadin fragments has been described elsewhere [4]. In brief, 10 mg of the recombinant γ-5 protein (prepared as described in the section above) was dissolved in 8 M urea/0.4 M NH₄HCO₃ and then reduced, alkylated and dialyzed against 0.1 M NH₄HCO₃/0.1 mM CaCl₂. Following digestion with α-chymotrypsin (1:100 wt/wt) the material was subjected to gelfiltration using a FPLC with a Superdex 200 HR 10/30 column (Amersham Pharmacia Biotech) in a 0.1 M NH₄HCO₃ buffer. Prior to testing for T cell recognition fractions were treated with 100 µg/ml guinea pig tTG (Sigma Chemical Co.) in 0.8 mM CaCl₂. Fractions containing stimulatory material were further separated by anion exchange chromatography (Mono-Q PC 1.6/5; Amersham Pharmacia Biotech) equilibrated with 5 mM Tris/HCl buffer, pH 6.5, and developed with a gradient with a final ending at 50 mM NaCl. T cell stimulatory MonoQ fractions were subsequently subjected to reverse-phase HPLC (µRPC C2/C18; Pharmacia) using a gradient running from 100% buffer A (0.1% TFA in H₂O) to 100% buffer B (80% acetonitrile, 19.9% H₂O, 0.1 % TFA). The Mono-Q and the reverse-phase HPLC were run on a SMART system (Pharmacia).

### Preparation of antigen.

Pepsin, pepsin-trypsin or chymotrypsin digestion of crude gliadin was performed as previously described [7, 8]. The peptides were either purchased from Research Genetics or synthesized at the Institute of Organic Chemistry, University of Tübingen, Germany. The latter synthetic peptides were prepared by multiple solid-phase peptide synthesis on a robotic system (Syro MultiSynTech, Bochum, Germany) using Fmoc/OtBu-chemistry and 2-chlorotrityl resin (Senn Chemicals AG, Dielsdorf, Switzerland) [9]. Identity of the peptides was confirmed by electrospray mass spectrometry and purity was analyzed by RP-HPLC. Treatment of the peptides with guinea pig tTG was performed in 37°C for 2 hours in PBS and 1 mM CaCl₂ using 100 µg/ml of tTG.

### Gliadin specific T cells.

T cell culturing and assays were performed in RPMI 1640 supplemented with 15% pooled, heat inactivated human serum, 0.01 M 2-ME, penicillin/streptomycin and 2.5 µg/ml Plasmocin (InvivoGen). The generation of T cells lines was performed as previously described [4]. In short, single biopsy specimens were cultured overnight in an organ culture chamber by immersion in culture medium with gliadin antigen. Biopsies from patients CD380, CD410, CD370, CD387, CD411 and CD430 were challenged with a pepsin-trypsin digest of gliadin, biopsies from patient CD436 were stimulated with a pepsin digest of gliadin, biopsies from patients CD377, CD421 and CD423 were stimulated with chymotrypsin digested gliadin and biopsies from the patient CD432, CD429 and CD450 were stimulated with chymotrypsin digested gluten. Gliadin from Sigma Chemical Co., gliadin extracted from flour prepared from the wheat strain Kadett, or gluten extracted from the wheat strains Avle or Mjolner were used as antigens. Following challenge, the biopsies were chopped with a scalpel and treated with collagenase A, or passed through a Medimachine (DAKO) to produce single cell suspensions, filtered through a 70 µm filter and seeded into 96 U-bottomed plates containing irradiated autologous PBMCs together with 10 U/ml IL-2. The cells were cultured in 5% CO₂ at 37°C.

TCC were established from antigen specific TCL by seeding at limiting dilution in a volume of 20 µl in the presence of 2x10⁴ allogeneic irradiated PBMC, 3 µg PHA and 10 U/ml IL-2. TCL and TCC were expanded by periodic stimulation with 3 µg/ml PHA, 10 U/ml IL-2 and allogeneic irradiated PBMC.

### Sequencing of T-cell clones.

For T cell receptor sequencing mRNA was isolated from the T cell clones. The mRNA was transcribed into cDNA and the T cell receptor V-alpha and V-beta gene usage was determined using V-alpha and V-beta specific primers. The relevant cDNA fragments were sequenced by the company BaseClear (Leiden, The Netherlands).

### RESULTS

### 1. Establishment of gluten specific T cell lines (TCL) from paediatric patients

In order to investigate the gluten specific T cell response early after disease induction, T cell biopsies were collected from patients that were suspected of celiac disease as indicated by either typical clinical symptoms and/or a positive anti-endomysium test. The age of patients at time of biopsy was between 1 year and 9 years. In the present study only patients with a confirmed diagnosis of celiac disease have been included. All patients expressed the disease associated DQ2 allele (DQA1*05/DQB1*02).

When multiple biopsies of a patient were obtained individual biopsies were cultured with either a trypsin/pepsin digest of gluten (termed gluten hereafter) or the same preparation that had additionally been treated with tTG (termed tTG-gluten hereafter). After 5 days IL-2 was added and cultures that showed evidence of T cell proliferation were expanded and tested for specificity in a proliferation assay using the two gluten preparations and HLA-DQ matched antigen presenting cells (see below). With one exception we only succeeded to grow gluten specific T cells from biopsies of patients that were diagnosed with celiac disease (not shown).

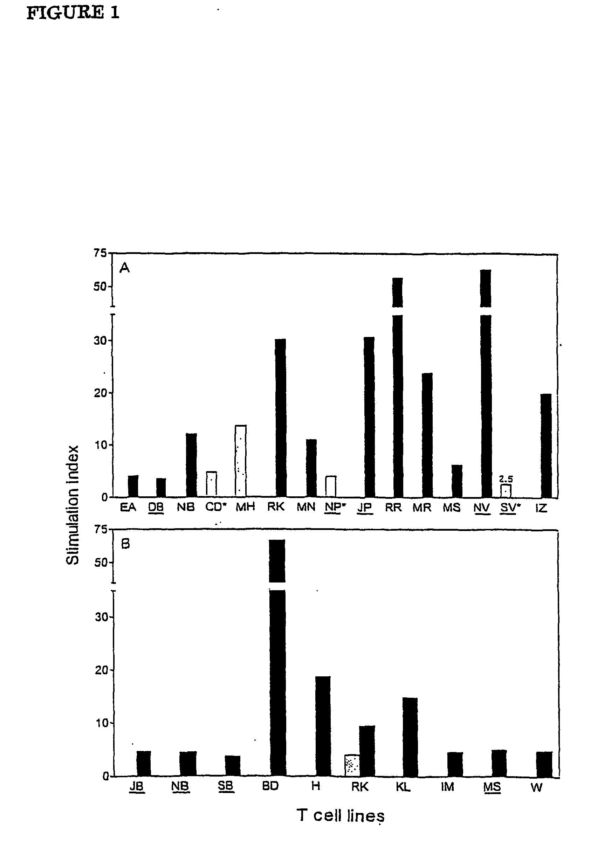

Gluten specific T cell lines were selected after initial stimulation with gluten or tTG-gluten (Figure 1). Altogether 26 gluten reactive T cell lines were obtained from 22 patients. Sixteen T cell lines were acquired after primary stimulation of the biopsies with gluten (Figure 1A). Out of these 16 lines only 5 responded to stimulation with gluten while the remainder responded to tTG-gluten (Figure 1A). Ten T cell lines were acquired after primary stimulation of the biopsies with tTG-gluten, all of which responded to stimulation with tTG-gluten whereas one also responded towards gluten (Figure 1B).

Thus, a large part of the gluten specific T cell response in paediatric patients appeared to be directed towards deamidated gluten but in 5 out of 22 patients (∼ 25%) a response towards non-deamidated gluten was also evident.

### 2. Generation of gluten specific T cell clones (TCC)

Gluten specific T cell clones were generated from gluten specific T cell lines of nine patients (Table 1). These clones were tested against gluten and tTG-gluten in the presence of HLA-DQ matched antigen presenting cells. Three patterns of reactivity were observed: i) T cell clones that did not respond to gluten but did respond to tTG-gluten (Table 1, tTG-gluten only); ii) T cell clones that responded to both gluten and tTG-gluten, tTG treatment often enhanced this reactivity (Table 1, gluten & tTG-gluten); iii) T cell clones that did respond to gluten but not to tTG-gluten (Table 1, gluten only). In 8 out of 9 patients tTG-dependent clonal T cell responses were found. In six patients, however, specific responses to non-deamidated gluten were also observed.

Thus in agreement with the results obtained with the polyclonal T cell lines a large proportion of the gluten specific responses is directed to deamidated gluten but responses to non-deamidated gluten are also common in paediatric patients.

**Table 1. Gluten specific T cell clones derived from polyclonal gluten specific T cell lines of children with celiac disease.**

| **Patient** | **Age (years)** | **HLA Typing** | **# T cell clones*** | **# T cell clones responding to** | | |
|---|---|---|---|---|---|---|
| | | | | **tTG-gluten only^{‡}** | **Gluten & tTG-gluten** | **gluten only** |
| DB | 6.1 | DQ2, DR3 | 11 | 5^{§} | 2 | 4 |
| JB | 3.1 | DQ2, DR3 DR7 | 10 | 8 | 1 | 1 |
| NBⁱ | 4.0 | DQ2, DR3 | 32 | 32 | - | - |
| SBⁱ | 4.0 | DQ2, DR3 | 28 | 24 | 4 | - |
| NP | 3.8 | DQ2, DR3 | 1 | - | 1 | - |
| JP | 1.2 | DQ2, DR3 | 34 | 14 | 10 | 10 |
| MS | 4.3 | DQ2, DR3 | 13 | 12 | 1 | - |
| NV | 1.2 | DQ2, DR3 DR7 | 18 | 18 | - | - |
| SV | 2.4 | DQ2, DR3 DR7 | 37 | 2 | 32 | 3 |

| | | | | | | |
|---|---|---|---|---|---|---|
| * # clones generated from polyclonal gluten reactive T cell line ^{‡} tTG treatment of gluten differentially affects clonal T cell responses to gluten: **tTG-gluten only:** T cell clones that respond to tTG-gluten only. **tTG- gluten and gluten:** T cell clones that respond to gluten and tTG-gluten, tTG often enhances T cell reactivity. **gluten only:** T cell clones that respond to gluten only. ^{§} Number of T cell clones exhibiting the gluten specific reactivity indicated ⁱ NB and SB are identical twins, the other patients are unrelated. | | | | | | |

**Table 2. Amino acid sequence of novel T cell stimulatory gluten peptides The amino acid sequence of four of the novel gluten epitopes could be matched with protein sequences from databases, and are named after the origin of the peptide: Glia-α, Glia-γ, and Glt, for α-gliadin, γ-gliadin and glutenin molecules respectively. The remaining two gluten epitopes are indicated with Glu. The amino acid sequence of the characterised peptides, the minimal epitopes required for T cell stimulation and the designation of the T cell clones (TCC) used to characterise the peptides, are indicated. The glutamine residues that are specifically deamidated by treatment with tTG are indicated in bold.**

| **Designation** | **Characterised peptide** | **Minimal epitope** | **T Cell Clone** |
|---|---|---|---|
| **Glia-α20**₍₉₃₋₁₀₆₎ | PFRPQQPYPQPQPQ | nd* | JB20 |
| **Glt**-**156**₍₄₀₋₅₉₎ | VQGQGIIQPQQPAQL | IIQPQQPAQ | SV30 |
| **Glia**-**γ30**₍₂₂₂₋₂₃₆₎ | QQQQPPFSQQQQSPFSQQ QQ | PFSQQQQSPF | MS156 |
| **Glt17**₍₄₆₋₆₀₎ | QQPPFSQQQQQPLPQ | PFSQQQQQ | NV17 |
| **Glu-21** | QPQPFPQQSEQSQQPFQP QPF | QSEQSQQPFQ PQ | SV21 |
| **Glu-5** | QQXSQPQXPQQQQXPQQP QQF^{‡} | QXPQQPQQF | JP437 and P27 |

| | | | |
|---|---|---|---|
| not determined ^{‡}X is isoleucine or leucine | | | |

### 3. Characterisation of novel gluten epitopes

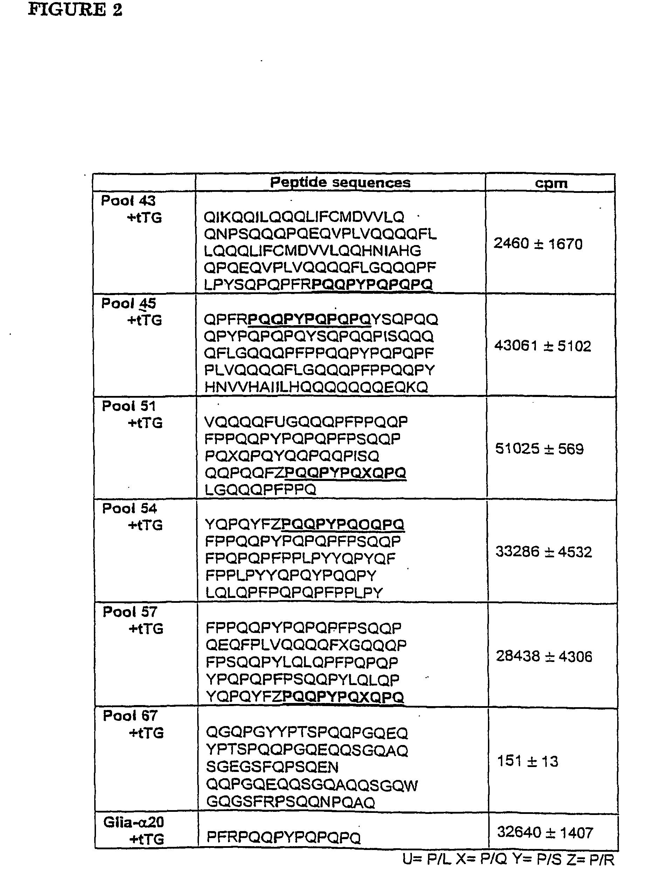

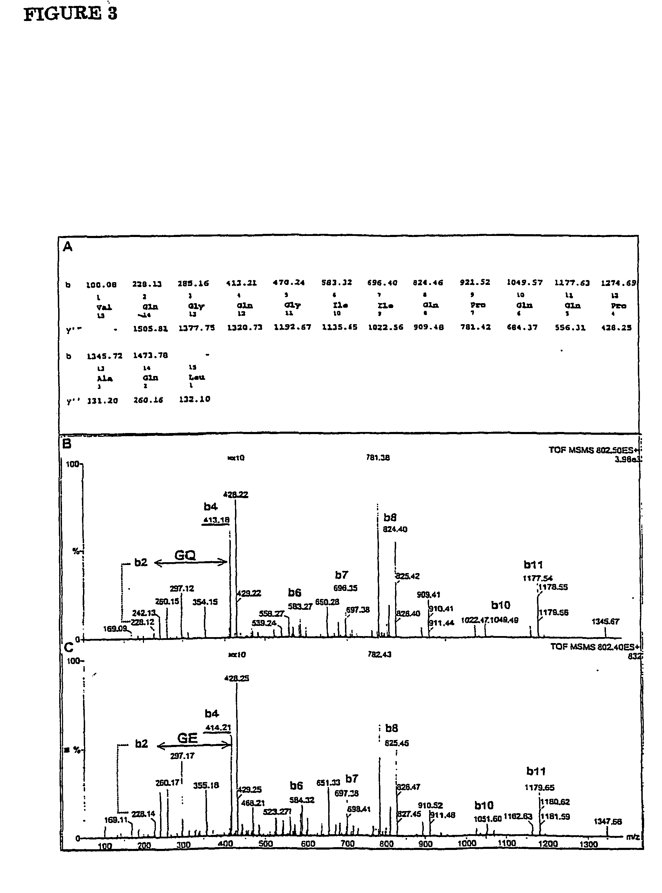

Next we determined the specificity of several of the gluten specific T cell clones. To this end the clones were first tested against peptides corresponding to three known HLA-DQ2 restricted T cell stimulatory gliadin derived peptides (see below). This analysis indicated that the large majority of the T cells did not respond to these peptides, and were thus likely to be reactive towards yet unidentified gluten peptides (not shown). To characterise these novel peptides we have used two different methods. First we identified gluten epitopes from a pepsin/trypsin digest of (tTG-) gluten. The digests were fractionated by repetitive rpHPLC and epitopes in the T cell stimulating fractions were identified by ESI-mass spectrometry as described [1, 2]. This method led to the characterisation of three novel T cell stimulatory peptides: Glia-γ30(222-236), Glu-21, and Glu-5 (Table 2). Second we tested the T cell clones against a set of 250 synthetic gluten peptides. The sequences, both gliadin and glutenin, were derived from gluten databases. Pools of 5 peptides, untreated and treated with tTG, were tested in a T cell proliferation assay. After identification of T cell stimulatory peptide pool(s) the individual peptides in that pool were analysed to identify the T cell stimulatory peptide. A representative example of this procedure is given in Figure 2. Clone JB20 responded towards 5 out of 50 peptide pools (Figure 2A). Analysis of the sequence of the peptides present in those pools indicated that the sequence PQQPYPQPQPQ was present in all the T cell stimulatory pools and thus likely responsible for the T cell stimulatory activity (Figure 2B). Testing of the individual peptides confirmed this (not shown). This method has led to the identification of three additional novel T cell stimulatory peptides: Glia-α20(93-106), Glt-156(40-59) and Glt-17(46-60) (Table 2). The latter two peptides, though distinct, show a large degree of sequence homology, e.g. they share the sequence QQPPFSQQQQ (see Table 2). For four of these six novel peptides deamidation by tTG either enabled or enhanced the T cell stimulatory activity (see below). Therefore, the effect of tTG treatment was determined by mass spectral analysis of the original peptides and the tTG treated peptides as described previously [3]. A representative example of this procedure is shown in figure 3. This analysis indicated that the glutamine residues underlined in Table 2 are modified by tTG.

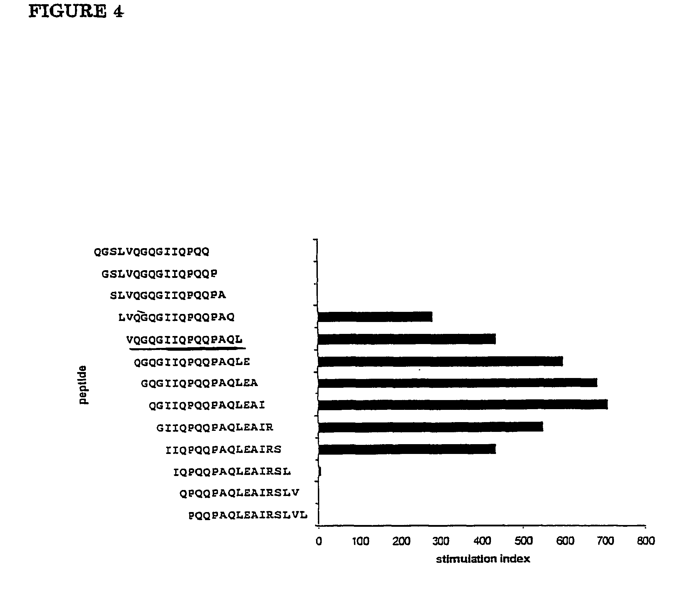

In order to identify the minimal peptide sequence required for the induction of T cell stimulation, N- and C-terminal truncation variants of these 5 peptides were synthesised and tested for their T cell stimulatory activity essentially as described before [1, 2]. A representative example of this procedure is shown in Figure 4. This analysis has led to the minimal epitopes indicated in Table 2.

### 4. Clonal analysis of T cell responses to novel gluten peptides demonstrates three modes of responses.

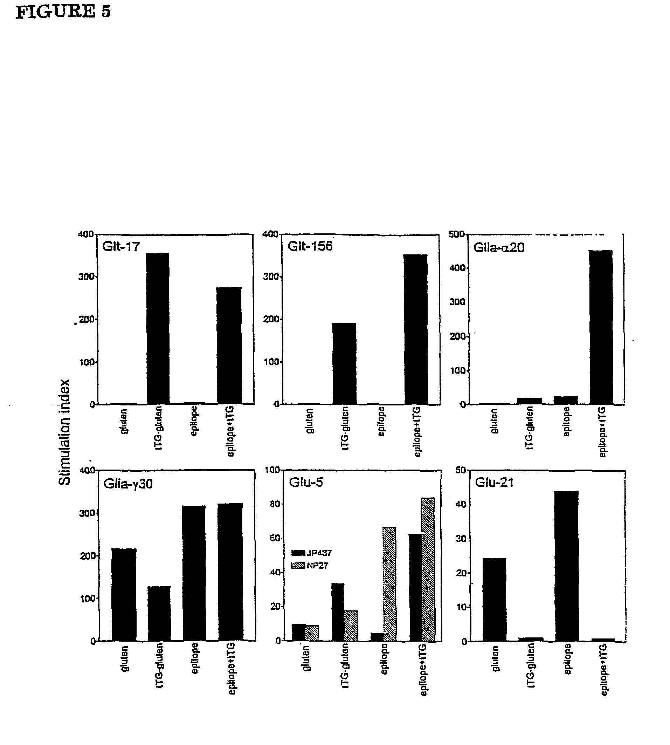

Subsequently we tested the response of the T cell clones to the identified peptides in deamidated and non-deamidated form (Figure 5). The response of T cell clone SV30 towards the Glia-γ30 peptide was found to be largely indifferent to deamidation. In contrast, the response towards the Glia-α20, Glt-17 and Glt157 peptides required prior deamidation. While the response towards the Glu-5 peptide is dependent on deamidation in the case of TCC JP43, it was not influenced by deamidation in the case of TCC NP27. Finally, deamidation abolished the response towards the Glu-21 peptide. Thus, in agreement with the results shown in Table 1, the effect of tTG on gluten specific T cell stimulation is heterogeneous and can be positive, neutral and negative.

### 5. T cell reactivity towards naturally occurring, variant peptide sequences

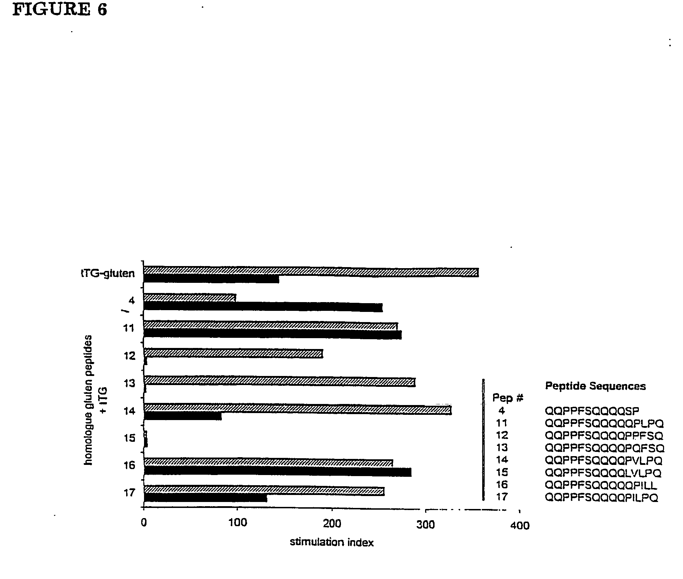

Homology searches in a dedicated gliadin/glutenin database indicated that the identified gliadin peptides represent relatively rare sequences (not shown). In contrast, many natural variants of the glutenin sequences were found. A search with the sequence QQPPFSQQQQ, which is shared between the T cell stimulatory glutenin peptides, yielded 95 hits in the gliadin/glutenin database (not shown). Further analysis revealed that this represented 34 distinct but homologous sequences. Of these, 32 were glutenin sequences while 2 were gliadin derived. Eight of these sequences were selected, synthesised and tested for T cell stimulatory activity (Figure 6). Five of the peptides were found to stimulate the Glt156 reactive T cell clone MS156, while the T cell clone NV17 responded to 7 of these peptides. Thus, the response of these glutenin specific T cell clones is highly promiscuous and directed to multiple glutenin homologous peptides. Strikingly, while each clone exhibited a unique reactivity pattern, both clones responded to stimulation with glutenin and gliadin derived homologues.

### 6. Heterogeneity in paediatric T cell responses towards gluten peptides

Subsequently the gluten specific T cell clones of all patients were tested against the previously characterised HLA-DQ2 restricted gluten peptides as well as against the peptides reported in the present study. The results obtained with representative clones from each patient are summarised in Table 3. Responses to some peptides were found in one patient only, while responses to the novel Glu-5 and Glia-α20 peptides as well as the previously identified gamma-gliadin peptide were found in various patients. These thus represent more immunodominant peptides in paediatric patients. T cell responses towards the α-gliadin peptides which have been reported to be immunodominant in adult patients were found in three paediatric patients, among whom the identical twins that show very similar reactivity against the gluten epitopes. Moreover, in these 9 patients we observed 8 different reactivity patterns as a consequence of tTG treatment of gluten (Table 3). These results indicate a highly diverse response against the various peptides.

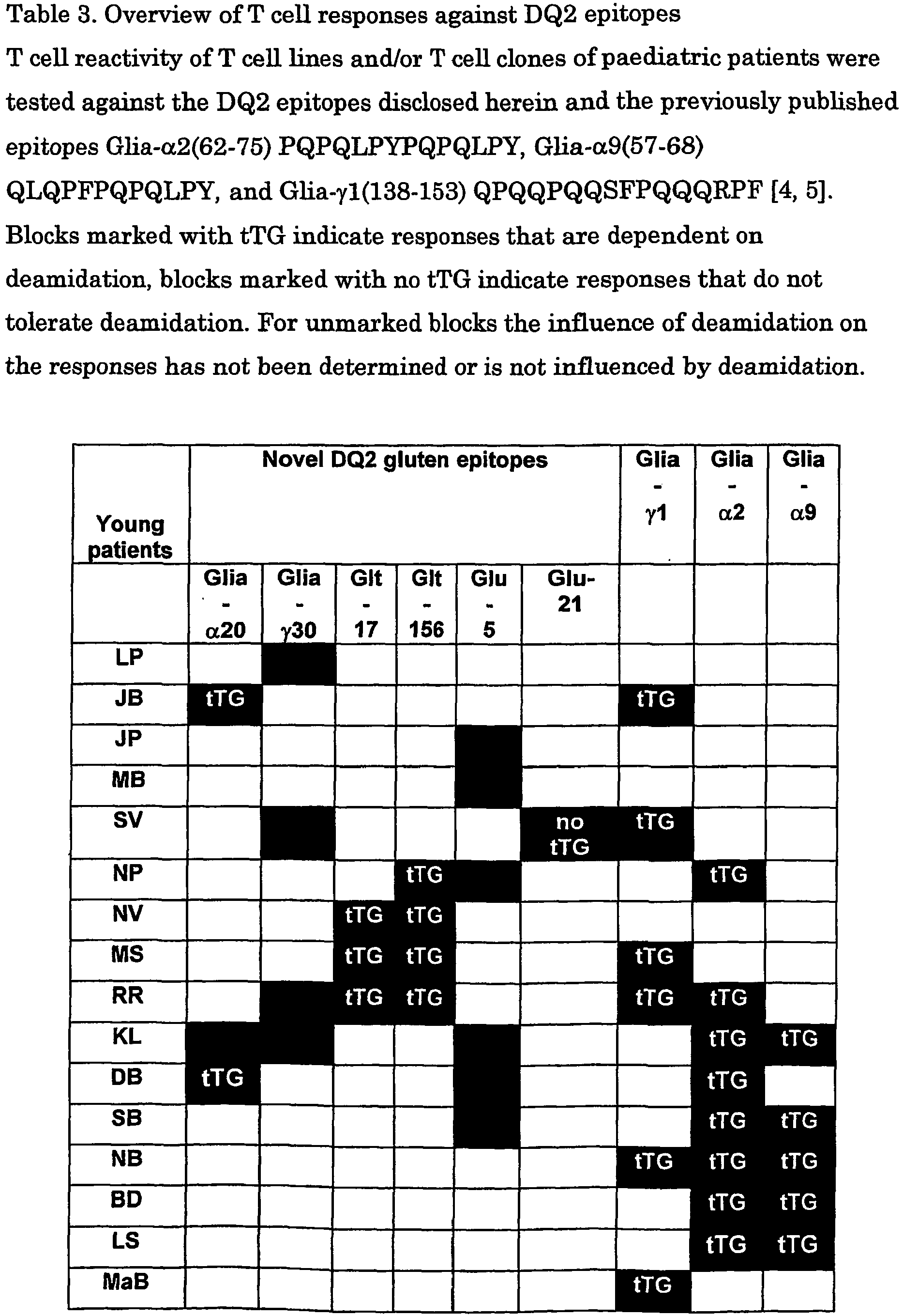

### 7. Amplification, cloning and sequencing of the γ-gliadin genes

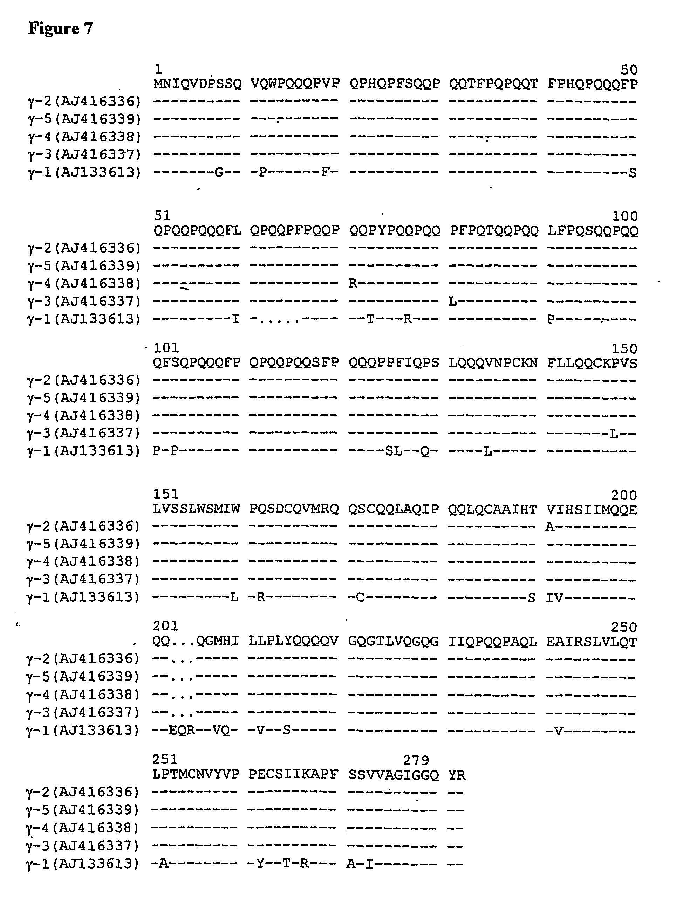

To express a panel of γ-gliadin genes genomic DNA from the wheat strain Mjølner (a wheat strain commonly grown in Norway) was isolated. PCR primers were designed to amplify all known mature γ-gliadins. Partial DNA sequencing of 29 independent clones obtained from a PCR with these γ-specific primers gave 12 sequences unrelated to gliadin and 17 gliadin sequences. Subsequent screening of the gliadin genes for expression identified 11 clones that could be productively expressed and purified. Sequencing of these gliadin genes identified 11 unique sequences. At the protein level these 11 distinct DNA sequences translated into 5 distinct γ-gliadins (γ-1, γ-2, γ-3, γ-4 and γ-5, respective accession numbers AJ133613, AJ416336, AJ416337, AJ416338 and AJ416339) (Figure 7) and all contained the known Glia-γ-1 and the Glia-γ-30 epitopes. By performing a BLAST search for the deduced protein sequences in all the major non-redundant protein sequence databases, only one of the recombinant γ-gliadins (γ-1) gave an identical match with a previous entry (GenBank accession number AJ133613).

### 8. Proteolytic fragments of recombinant γ-5 gliadin stimulatory for intestinal T cells

As gliadin proteins are insoluble at physiological salt concentrations, the recombinant γ-gliadins were made soluble by digestion with either pepsin or chymotrypsin prior to use in T cell assays. These soluble antigens were then treated with tissue transglutaminase and tested for their ability to stimulate a panel of gluten specific TCL that we had previously found not to respond to either of the known γ-gliadin epitopes (Glia-γ-1 and Glia-γ-30) (Table 4). Initially, we found that a T cell line from the patient CD411 (TCL CD411E) responded to all the tTG-treated recombinant gliadin proteins (γ-1 to γ-5), but had no or only a low response to the same gliadins untreated by tTG. As this T cell line did not recognize any of the known γ-gliadin epitopes (Glia-γ-1 and Glia-γ-30) this indicated that the response was towards new identified peptide epitopes in the γ-gliadin.

**Table 4. Screening of intestinal T cell lines against a panel of recombinant γ-gliadins**

| TCL | + | γ-1 | γ-2 | γ-3 | γ-4 | γ-5 |
|---|---|---|---|---|---|---|
| 380 E | 29.9 | 1.7 | 1.54 | 1.9 | 1.4 | 1.1 |
| 377.5 | 7.4 | 1.2 | 1.1 | 1.3 | 1.5 | 1.2 |
| 411 E | 17.5 | 3.2 | 2.1 | 5.8 | 7.2 | 6.6 |
| 421.1.1 | 18.9 | 0.9 | 0.9 | 1.3 | 0.7 | 0.7 |
| 430 1.d | 23.6 | 1.7 | 1.7 | 1.5 | 1.9 | 1.9 |
| 410 | 12.8 | 1.4 | 0.6 | 1.2 | 1.6 | 0.6 |

| | | | | | | |
|---|---|---|---|---|---|---|
| *T cell lines (TCL) isolated from six adult CD patients were tested for recognition of 5 different chymotrypsin digested and tTG treated recombinant γ-gliadins (γ-1 to γ-5). TCLs that did not respond to any of the two previously characterized γ-gliadin epitopes DQ2-γ-I [5] and DQ2-γ-II were chosen. Chymotrypsin digested gliadin from the wheat variety Kadett was used as a positive control* (+). *Results are given as the stimulation index (SI), calculated with help of the next formula: (cpm after specific stimulation - cpm background)*/*cpm background.* | | | | | | |

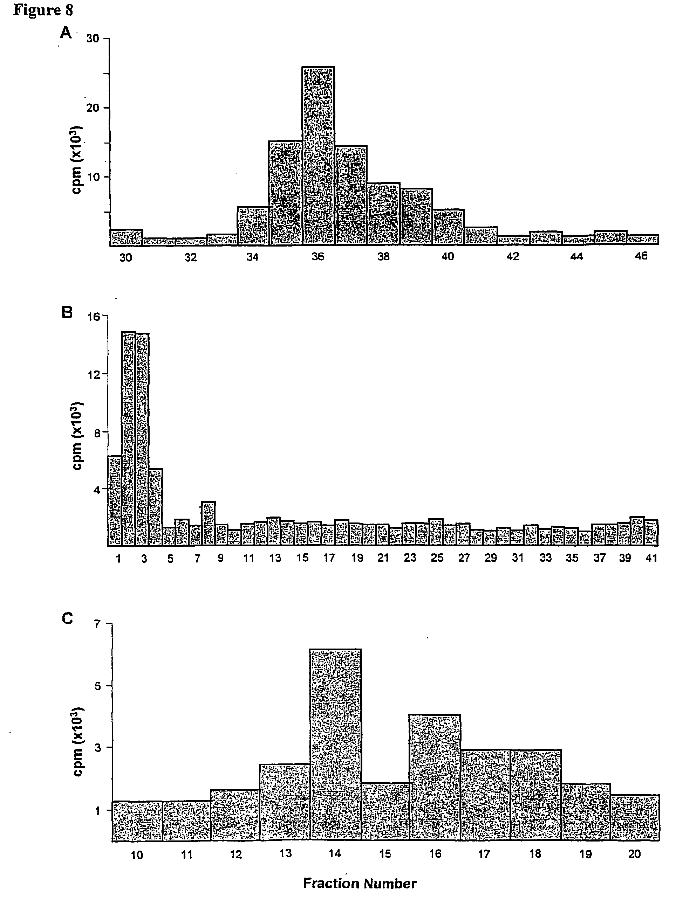

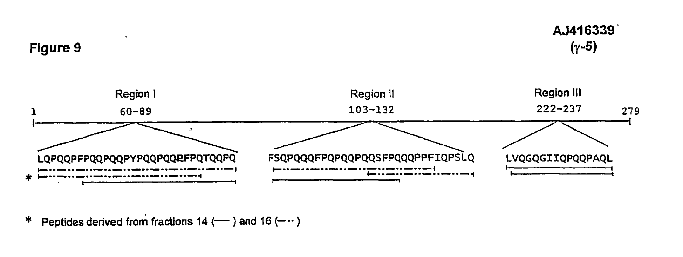

To identify the T cell reactive epitopes in γ-gliadin, peptide fragments were isolated from one of the recombinant gliadins (γ-5) following series of biochemical purification steps using two T cell clones made from the T cell line CD411E to identify positive fractions (TCC CD411 E2.47 and TCC CD411 E2.104, referred to as TCC 411A and TCC 411C, respectively). The γ-5 recombinant gliadin was treated with chymotrypsin and separated using size exclusion chromatography (Superdex 200 HR 10/30 column). Fractions were then treated with guinea pig tTG and tested for recognition by TCC 411A (Figure 8A) and TCC 411C. Fraction 36 most efficiently stimulated the T cell clones and was subsequently subjected to ion exchange chromatography (Mono-Q PC 1.6/5). Notably, only a small proportion bound to the column whereas most of the material was found in the "flow-through" (fraction 2, 3 and 4). Nevertheless, as active material was found in these early fractions (Figure 8B), we applied the T cell reactive MonoQ fraction 2 to the reverse-phase HPLC (µRPC C2/C18). This produced two fractions (fraction 14 and 16) that stimulated the TCC 411A (Figure 8C). Fraction 16 also stimulated the TCC 411C. Analysis of these fractions by Electrospray ionization mass spectrometry (ESI) identified 8 different peptides clustered in three different regions of the γ-5 recombinant (Figure 9). Interestingly, fraction 14 contained peptides that overlapped completely the Glia-γ-30 epitope and partly the Glia-γ-1 epitope whereas fraction 16 contained peptides that completely overlapped the Glia-γ-1 epitope.

### 9. Identification of 3 new DQ2 restricted T cell epitopes in the γ-5 gliadin

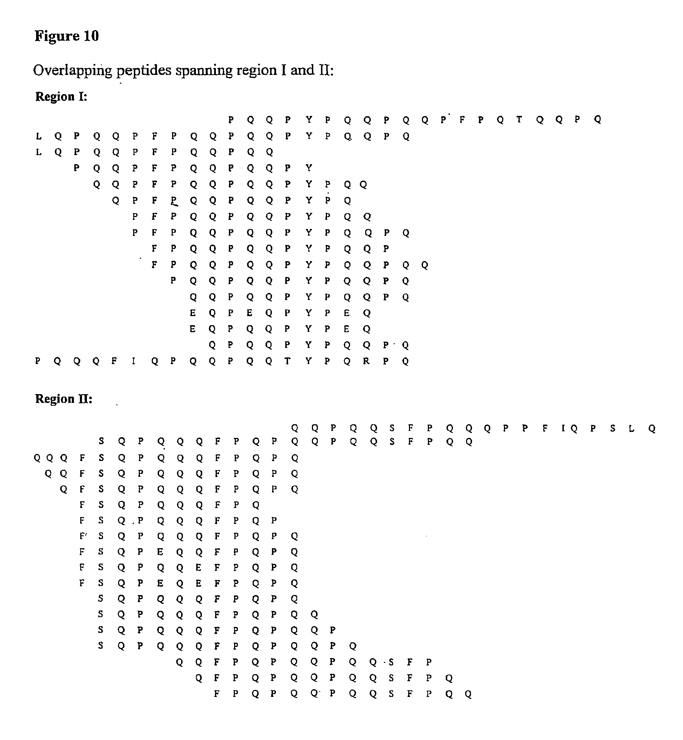

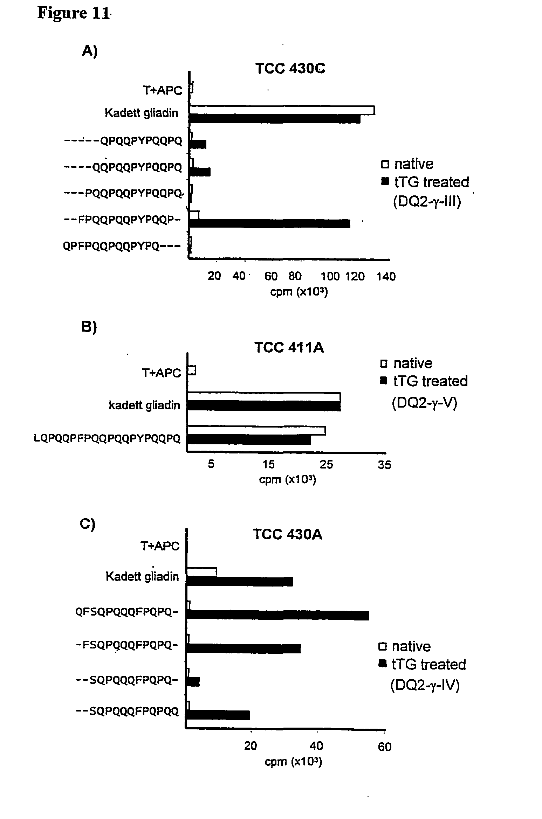

To identify the T cell epitopes contained within the HPLC fractions 14 and 16 of the γ-5 gliadin, overlapping peptides spanning the regions I (16 peptides) and II (18 peptides) (Figure 10) were synthesized. These peptides were tested against five TCC; TCC 411A and B and TCC 430 A, B and C. The latter three TCC were generated from an intestinal T cell line (TCL CD430) that was responsive to several peptides from region I and II. Two types of T cell reactivity patterns were found against peptides from region I. The first type of reactivity pattern is exemplified by the TCC 430B and TCC 430C. These TCC were reactive with the minimal peptide γ5 (66-78) (defined as the DQ2-γ-III epitope; Table 5A) in a strict tTG dependent manner (Figure 11A).

The TCC 411A and TCC 411B represent the second type of reactivity pattern against peptides of region I. These TCC recognized the peptide γ-5 (60-79) (defined as the DQ2-γ-V epitope; Table 5A), and for these TCC deamidation by tTG had no influence on T cell recognition, neither for the chymotrypsin treated crude gliadin nor for the peptide (Figure 11B).

A single type of reactivity pattern, represented by TCC 430A, was found against peptides of region II. This TCC recognized the peptide γ-5(102-113) (defined as the DQ2-γ-IV epitope: Table 5A) in a strictly tTG dependent manner, and the TCC had a weak response that was strongly enhanced by tTG treatment against chymotrypsin treated crude gliadin (Figure 8C).

### 10. Identification of a third α-gliadin epitope that clusters with the DQ2-α-I and DQ2-α-II epitopes

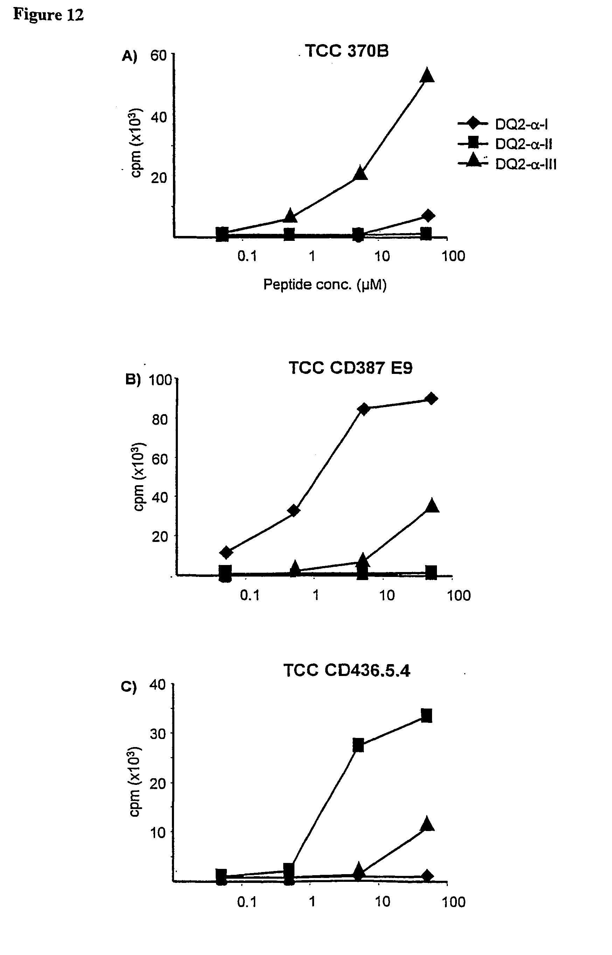

During the screening of T cells recently generated within our laboratory it became clear that a third α-gliadin epitope existed within the α-2 recombinant gliadin (accession number AJ133612). Two T cell clones were identified that were stimulated by the α-2 recombinant gliadin but failed to respond to either of the DQ2-α-I or DQ2-α-II peptide epitopes. Because the pattern of epitope clustering observed with the DQ2-α-I or DQ2-α-II epitopes was also evident with the epitopes in the γ-5 recombinant, we wondered whether the DQ2-α-III epitope also clustered with the DQ2-α-I and DQ2-α-II epitopes. Indeed, testing of peptide (α-2(64-75)) with Q→E substitution in position 72 and partly overlapping with both the DQ2-α-I and DQ2-α-II epitopes efficiently stimulated both the TCC CD370.2.25 and TCC CD370 E3.19 (referred to as TCC 370A and TCC 370B) (Figure 12A) whereas the DQ2-α-I and DQ2-α-II did not. Testing this native peptide failed to stimulate these two T cell clones. The glutamine in position 72 is naturally targeted by tTG and is located in the same position within the repetitive seven-residue fragment as for the two other epitopes. Testing of this new peptide (α-2(64-75)E72/DQ2-α-III (Table 5A) against the DQ2-α-I specific TCC CD387 E9 and the DQ2-α-II specific TCC CD436.5.4 elicited a low T cell response, and then only at a very high peptide concentration (50 µM), indicating that this epitope is distinct from the DQ2-α-I and the DQ2-α-II epitopes (Figure 12B and 12C). Table 5. Sequences given under A are new epitopes identified with T cell clones and have been characterised by fragments/peptides from the recombinant gamma-5 protein and a panel of synthetic peptides. The peptides disclosed under B are synthetic peptides from the M36999 gamma-gliadin which stimulate one or more T cell lines. The underlined parts show the deduced minimal epitopes.

**Table 5A: DQ2 restricted gliadin epitopes**

| Epitope | Peptide | Sequence¹ | T cell clones (TCC)/ T cell lines (TCL) | |
|---|---|---|---|---|
| DQ2-γ-III | γ-5 (66-78)E68,E71 | FPQQPQQPYPQQP² | TCC: | 430B, 430C |
| | | | | |
| DQ2-γ-IV | γ-5 (102-113)E106,E108 | FSQPQQQFPQPQ³ | TCC: | 430A |
| | | | TCL: | CD411 E, CD429.1.6 |
| DQ2-γ-V | γ-5 (60-79) | | | |
| | | LQPQQPFPQQPQQPYPQQPQ | TCC: | 411A, 411B |
| | | | TCL: | CD411 E |
| DQ2-α-III | α-2 (64-75)E72 | PQLPYPQPQLPY | TCC: | 370A, 370B, |
| | | | TCL: | CD419.3, CD411 E, CD433.1, CD380 E3 |

**Table 5b: γ-gliadin-derived dodecapeptide epitopes recognized by T cells.**

| Epitope | Designation | Peptide sequence^{4,5} | Homology to epitopes | T cell clones (TCC)/ T cell lines (TCL) |
|---|---|---|---|---|
| M2 | M36999 (11-30) | WPQQQPFPQPQQPFCQQPQR | DQ2-α-I | TCL: CD411E, CD432.1.2 |
| M7 | M36999 (61-80) | QFPQTQQPQQPFPQPQQTFP | DQ2-α-I, DQ2-γ-IV | TCL: CD411E, CD432.1.2 |
| | | QFPQTQQPQQPFPQPQQTFP | | |
| M8 | M36999 (71-90) | PFPQPQQTFPQQPQLPFPQQ | DQ2-γ-III | TCL: CD411E |
| M10 | M36999 (91-110) | PQQPFPQPQQPQQPFPQSQQ | DQ2-α-I | TCL: CD411E |
| M12 | M36999 (111-130) | PQQPFPQPQQQFPQPQQPQQ | DQ2-γ-IV | TCL: CD411E, CD432.1.2, CD429.1.6 |

| | | | | |
|---|---|---|---|---|
| ¹ Glutamine residues targeted by tTG are in bold ² The MS/MS analysis indicated tTG-mediated deamidation of glutamines in position 3 and positions 6 or 7. ³ tTG-mediated deamidation of the glutamines in positions 106 and 108 is required for T cell recognition. ⁴ Sequences homologous to epitopes in Table 5a are underlined. ⁵ Glutamine residues targeted by tTG are not determined | | | | |

### 11. Epitopes identified in the γ-gliadin M36999 using overlapping peptides

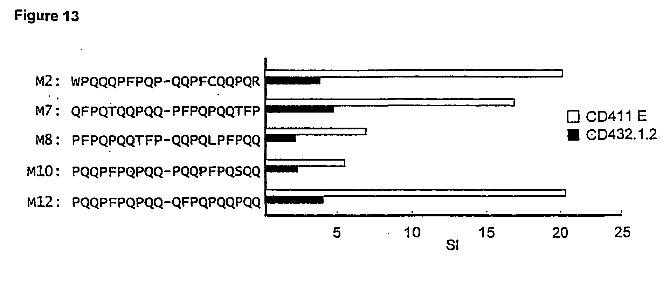

We also tested a set of 23x20mer peptides that overlapped by 10 residues and that cover nearly all of the gamma gliadin M36999 [10] and screened for recognition of these peptides after tTG treatment by a panel of 6 gluten specific polyclonal T cell lines. Five of these T cell lines made a response to the gamma gliadin derived peptides: the T cell line from patient CD411 (TCL CD411E) made a strong response against the peptides M2, M7 and M12 and a weaker response towards the peptides M8, M10 and M13 (Figure 13, Table 5B). Moreover, the T cell line from patient CD432 (TCL CD432.1.2) made a response to the peptides M2, M7 and M12 (Figure 13, Table 5B), whereas the T cell line from patient CD450 (TCL CD450.2.2) only made responses to the peptides M90 and M91, which includes the sequence of the DQ2-γ-II epitope. The TCL CD429.1.6 made a response to the M12 peptide and the TCL CD423.1.3 made a response to the M13 peptide, which includes the sequence of the DQ2-γ-I epitope. Peptides M2 and M7 contain sequences that are remarkably similar to DQ2-α-I epitope. Furthermore, the peptide M7 also includes sequences that are very similar to the DQ2-γ-IV epitope, as does the peptide M12. The latter differ from the DQ2-γ-IV epitope by only a single S to P substitution. These sequence similarities probably cause some degree of cross reactivity and likely the peptides M2, M7 and M12 harbor novel epitopes that bear similarities with other T cell epitopes.

### 12. Cross reactivity between T cell lines isolated from adult celiac disease patient and the novel peptides identified with the T cell clones from paediatric patient and vice versa.

T cell lines isolated from small intestinal biopsies of 22 adult celiac disease patients were tested against the novel gluten peptides that were identified with the T cell clones from children with celiac disease. Nine of these T cell lines responded to these peptides. In particular reactivity was observed against the Glia-alpha2, the Glia-gamma30, and Glu-5 peptides but not against the Glt-17, Glt-156 and Glu-21 peptides.

T cell lines isolated from small intestinal biopsies of 16 children with celiac disease were tested against Glia-alpha2 and Glia-alpha9 peptides previously identified [4]. Eight of these T cell lines responded to either one or both of these peptides.

These data indicate that some prolamine-derived peptides are only recognized by T cell clones/lines derived from adult or pediatric CD patients and that other prolamine-derived peptides are recognized by both groups of patients. This observation is for example used for the development of a sensitive diagnostic method based on the herein disclosed prolamine-derived peptides and their occurrence in the different patient groups.

### 13. CDR3 amino acid sequences of T cell receptor of selected gluten specific T-cell clones.

From 5 of the 7 T-cell clones depicted in Table 2, the CDR3 amino acid sequence of T-cell receptor of selected gluten specific T-cell clones was determined. The T-cell receptor Vα and Vβ gene usage was determined using Vα and Vβ specific primers. The results are depicted in Table 6.

For T-cell clone MS156 five distinct but clearly related CDR3 sequences were found. For clones SV30, SV21 and P27 only the amino acid sequence of the T-cell receptor β-chain has been determined.

**Table 6: CD3 amino acids sequences of T-cell receptors of selected gluten specific T-cell clones. Shown are the known Vα and Vβ gene segments used and the determined amino acid sequence of the CDR3 region and the designation of the J-element used.**

| **T cell clone** | **T-cell receptor V-gene used** | | **NDN¹** | | **J-region used** |
|---|---|---|---|---|---|
| MS156 | | | | | |
| | AV23S1 | CA | VPQ | ETSGSRLTFGEGTQLTVNPD | AJ58 |
| | BV6 | CASS | IRQ | GNTIYFGEGSWLTVV | BJ1S3 (original) |
| | | CASS | LYW | SSYEQYFGPGTRLTVT | BJ2S7 (variant) |
| | | CASS | FGAGGQK | YNEQFFGPGTRLTVL | BJ2S1 (variant) |
| | | CASS | LYW | SSYEQYFGPGTRLTVT | BJ2S7 (variant) |
| | | CASS | LASASGEY | TQYFGPGTRLTVL | BJ2S3 (variant) |
| | | | | | |
| JP437 | | | | | |
| | AV1S1 | CAV | NV | GGATNKLIFGTGTLLAVQPN | AJ32 |
| | BV21S3 | CASSL | FGGI | TDTQYFGPGTRLTVL | BJ2S3 |
| | | | | | |
| P27 | | | | | |
| | BV13S3 | CASSE | GQSGS | EAFFGQG | BJ1S1 |
| | | | | | |
| SV21 | | | | | |
| | BV4S1 | CSV | SVGQ | QETQYFGPG | BJ2S5 |
| | | | | | |
| SV30 | | | | | |
| | BV13S3 | CAS | TIQGG | ETQYFGPG | BJ2S5 |

| | | | | | |
|---|---|---|---|---|---|
| ¹ nucleotide insertion D-segment nucleotide insertion | | | | | |

### FIGURES

**Figure 1****. Gluten specific responses of T cell lines from paediatric celiac disease patients**
   Gluten recognation of small intestinal T cell lines generated after initial gluten challenge with gluten (A), and with tTG-gluten (B). The T cell lines were selected for recognition of gluten (shaded bars), and/or tTG-gluten (black bars) Responses were considered positive when the gluten specific stimulation was three times above background: SI ≥ 3. The T cell lines that were used for cloning are underlined. * Lines that were not tested against tTG-gluten.
**Figure 2****. Identification of the gliadin epitope Glia-α20 for TCC JB20**
   The Glia-α20 epitope is characterised by testing the response of the T cell clones against 50 peptide-pools ± tTg, each containing 5 gliadin and/or glutenin peptides. Five pools were recognised by TCC JB20. Comparison of the sequence of the peptides indicated a single sequence (underlined) that was present in the stimulatory pools but not in non-stimulatory pools, for example pool 67. This was confirmed by T cell recognition of a newly synthesised version of this peptide, termed Glia-α20. Indicated is raw cpm value (medium value 312 ± 324).
**Figure 3****. Mass spectral analysis of deamidation of the Glia-γ30 epitope**
   (A) Expected fragment ion masses of the Glia-γ30 epitope based on amino acid sequence. (B) Observed fragments of the Glia-γ30 epitope, b-ions are indicated according to panel A. (C) Observed fragments of the Glia-γ30 epitope after deamidation by tTG (C). The mass difference between the b-ions 228 and 413 in panel B, and between 228 and 414 in panel C correspond to the sequence GQ and GE respectively, indicating the Q to E conversion at position 4. Similarly, the Q at position 10 is converted in to an E by tTG treatment as indicated by the 2 Da shift of the b10-ion from 1049 to 1051.
**Figure 4****. Determination of the minimal epitope for Glia-γ30**
   Minimal epitopes were determined through testing of overlapping peptides that were based on sequence of the source protein of the Glia-γ30 peptide. This figure represents the T cell response of TCC SV30 against the originally identified peptide (underlined), and responses against the overlapping peptides. The minimal required sequence for induction of T cell proliferation is IIQPQQPAQ.
**Figure 5****. Recognition of the novel DQ2 epitopes**
   The effect of deamidation on recognition of the new gluten epitopes by T cell clones shows three major patterns. For T cell recognition of gluten epitopes Glt-17(46-60), Glt-156(40-59), and Glia-α20(93-106) tTG treatment is required. Equal or enhanced responses after specific deamidation by tTG are found for epitopes Glia-γ30, and Glu-5. In the third pattern the T cell reaction against the Glu-21 epitope is blocked by deamidation of the peptide, this epitope however contains a natural glutamic acid residue that provides a negative charge for potential binding to HLA DQ2.
**Figure 6****. Responses of two T cell clones against homologue peptides**
   Homology searches with a partial sequence of the Glt-156 epitope (QQPPFSQQQQ) yielded 34 unique matches in the gluten database. The T cell response against eight of these homologue gluten peptides was determined and found to be distinct for different T cell clones. TCC NV17 responds against all peptides except peptide 15, whereas TCC MS156 does not recognise peptides 12, 13 and 15.
**Figure 7** **Amino acid sequence alignment of the γ-gliadin clones γ-1 to γ5**
   The EMBL accession numbers of the DNA sequence and the clone names are indicated. A consensus amino acid sequence is given above the alignment. The N-terminal M and the C-terminal Y and R are non-gliadin sequences that are introduced as part of the expression vector. The sequences of the 6 N-terminal residues and the 8 C-terminal residues are determined by the primers used for the PCR-amplification.
**Figure 8** **Biochemical purification of peptide fragments stimulatory for the TCC 411A from the γ-5 recombinant gliadin**
   The T cell reactive Superdex fraction 36 of the tTG-treated γ-5 recombinant chymotrypsin-digest (A) was separated by ion exchange chromatography. MonoQ fraction 2 contained active material (B) and was further separated by reverse-phase HPLC. Both fractions 14 and 16 produced a small T cell stimulatory peak (C) and were subjected to ESI mass spectrometry. T cell responses are given in cpm.
**Figure 9** **Eight peptide fragments were identified with ESI mass spectrometry on reverse-phase HPLC fractions 14 and 16**
   These peptides cluster in three different regions within the γ-5 recombinant gliadin and are indicated below the sequence excerpts.
**Figure 10** **Overlapping synthetic peptides spanning region I and II of the recombinant γ gliadin**
   T cell epitopes in γ-5 were identified by testing overlapping synthetic peptides spanning the regions I (16 peptides) and II (18 peptides) against TCC derived from the patients CD411 and CD430.
**Figure 11** **Reactivity of T cell clones specific for DQ2-γ-III, DQ2-γ-V or DQ2-γ-I epitopes**
   Testing of truncated variants of the A) DQ2-γ-III and C) DQ2-γ-IV epitopes for their ability to stimulate the TCC 430C and TCC 430A, respectively. For the B) DQ2-γ-V epitope none of the shorter truncation variants stimulated the TCC 411A. The peptides were tested in its native form (white bars) or after treatment with guinea pig tTG (black bars). Peptides were tested at 10 µM. The responses are given in cpm.
**Figure 12** **Reactivity of three T cell clones, each specific for either the DQ2-α-I, DQ2-α-II or the DQ2-α-III epitope**
   Testing of peptide α-2(64-75) with Q→E substitution in position 72 (defined as DQ2-α-III) and partly overlapping with both the DQ2-α-I and DQ2-α-II epitopes efficiently stimulated the TCC 370B whereas the DQ2-α-I and DQ2-α-II did not (A). Testing of the DQ2-α-III epitope against the DQ2-α-I specific TCC CD387 E9 (B) and DQ2-α-II specific TCC CD436.5.4 (C). Responses are given in cpm.
**Figure 13** **T cell recognition of some tTG treated peptides derived from the M36999 γ-gliadin**
   Testing of the peptides M2, M7, M8, M10 and M12 against the T cell lines CD411 E and CD432.1.6. Results are given as the stimulation index, calculated by the dividing the proliferative response to antigen by the background (T+APC). Peptides were tested at 10 µM.

### REFERENCES

1. Wal van de, Y., Kooy, Y.M.C., Veelen, van P., August, S.A., Drijfhout, J.W. and Koning,F.Glutenin is involved in the gluten-driven mucosal T cell response.Eur. J. Immunol. 29, 3133-3139 (1999).
2. Wal van de Y, Kooy Y, Veelen van P, Pena S, Mearin L, Molberg Q, Lundin L, Mutis T, Benckhuijsen W, Drijfhout J.W, and Koning F. Small intestinal cells of celiac disease patients recognize a natural pepsin fragment of gliadin. Proc. Natl. Acad. Sci. USA. 95, 10050-10054 (1998)
3. Wal van de Y, Kooy Y, Veelen van P, Pena S, Mearin L, Papadopulos G, and Koning F. Cutting Edge: Selective deamidation by tissue transglutaminase strongly enhances giadin-specific T cell reactivity. J. Immunol. 161, 1585-1588 (1998)
4. Arentz-Hansen,H., R.Korner, O.Molberg, H.Quarsten, W.Vader, Y.M.Kooy, K.E.Lundin, F.Koning, P.Roepstorff, L.M.Sollid, and S.N.McAdam. 2000. The intestinal T cell response to alpha-gliadin in adult celiac disease is focused on a single deamidated glutamine targeted by tissue transglutaminase. J.Exp.Med. 191:603-612.
5. Sjostrom,H., K.E.Lundin, O.Molberg, R.Korner, S.N.McAdam, D.Anthonsen, H.Quarsten, O.Noren, P.Roepstorff, E.Thorsby, and L.M.Sollid. 1998. Identification of a gliadin T-cell epitope in coeliac disease: general importance of gliadin deamidation for intestinal T-cell recognition. Scand.J.Immunol. 48:111-115.
6. Arentz-Hansen,E.H., S.N.McAdam, O.Molberg, C.Kristiansen, and L.M.Sollid. 2000. Production of a panel of recombinant gliadins for the characterisation of T cell reactivity in coeliac disease. Gut 46:46-51.
7. Lundin,K.E., H.Scott, T.Hansen, G.Paulsen, T.S.Halstensen, O.Fausa, E.Thorsby, and L.M.Sollid. 1993. Gliadin-specific, HLA-DQ(alpha 1*0501,beta 1*0201) restricted T cells isolated from the small intestinal mucosa of celiac disease patients. J.Exp.Med. 178:187-196.
8. Molberg,O., S.N.McAdam, R.Korner, H.Quarsten, C.Kristiansen, L.Madsen, L.Fugger, H.Scott, O.Noren, P.Roepstorff, K.E.Lundin, H.Sjostrom, and L.M.Sollid. 1998. Tissue transglutaminase selectively modifies gliadin peptides that are recognized by gut-derived T cells in celiac disease. Nat.Med. 4:713-717.
9. Jung,G. 1996. Combinatorial peptide and nonpeptide libraries - A handbook for the search of lead structures. In Combinatorial peptide and nonpeptide libraries - A handbook for the search of lead structures. G.Jung, editor. Verlag Chemie, Weinheim.
10.Scheets,K. and C.Hedgcoth. 1988. Nucleotide sequence of a gamma-gliadin gene: Comparisons with other gamma-gliadin sequences show the structure of gamma-gliadin genes and the general primary structure of gamma-gliadins. Plant Science 57:141-150.

## Claims

1. An isolated, recombinant or synthetic peptide comprising the amino acid sequence PFRPEQPYPQPQPQ.

2. An isolated, recombinant or synthetic peptide according to claim 1, wherein said peptide is flanked by amino acids representing antigen processing sites.

3. An antibody specifically recognizing the amino acid sequence PFRPEQPYPQPQPQ in a peptide according to claim 1 or claim 2.

4. An immunoassay comprising an antibody according to claim 3.

5. A diagnostic kit comprising
- an antibody according to claim 3 and
- a suitable means of detection.

6. A diagnostic kit according to claim 5 for detecting in food, food components or biological samples the presence of a prolamine-derived peptide involved in food-related immune enteropathy.

7. A diagnostic kit according to claim 6 wherein said immune enteropathy is selected from the group of celiac sprue, tropical sprue, giardiasis or food allergies of childhood.

8. A method to decrease the amount of toxic prolamine-derived peptides in food or food components comprising incubating an antibody according to claim 3 with said food or food component.

9. A method to select a cereal comprising providing an antibody according to claim 3 and selecting said cereal on basis of reactivity.

10. A pharmaceutical composition comprising a peptide according claim 1 or claim 2.

11. A pharmaceutical composition according to claim 10 for the induction of tolerance.

12. A pharmaceutical composition according to claim 10 for the treatment of gluten-sensitivity.

13. A pharmaceutical composition according to claim 10 for the elimination of gluten-sensitive T-cells.

## Patentansprüche

1. Isoliertes rekombinantes oder synthetisches Peptid, umfassend die Aminosäuresequenz PFRPEQPYPQPQPQ.

2. Isoliertes rekombinantes oder synthetisches Peptid nach Anspruch 1, wobei das Peptid von Aminosäuren, die Antigen-verarbeitende Stellen darstellen, flankiert ist.

3. Antikörper, der spezifisch die Aminosäuresequenz PFRPEQPYPQPQPQ in einem Peptid nach Anspruch 1 oder Anspruch 2 erkennt.

4. Immuntest, umfassend einen Antikörper nach Anspruch 3.

5. Diagnose-Kit, umfassend
- einen Antikörper nach Anspruch 3 und
- ein geeignetes Detektionsmittel.

6. Diagnose-Kit nach Anspruch 5, um in Lebensmitteln, Lebensmittelkomponenten oder biologischen Proben die Präsenz eines von einem Prolamin abstammenden Peptids, das an der lebensmittelbezogenen Immun-Enteropathie beteiligt ist, festzustellen.

7. Diagnose-Kit nach Anspruch 6, wobei die Immun-Enteropathie aus der Gruppe von Zöliakie, tropischer Sprue, Giardiasis oder Lebensmittelallergien bei Kindern ausgewählt ist.

8. Verfahren zur Verminderung der Menge an toxischen, von Prolamin abstammenden Peptiden in Lebensmitteln oder Lebensmittelkomponenten, umfassend die Inkubation eines Antikörpers nach Anspruch 3 mit einem Lebensmittel oder einer Lebensmittelkomponente.

9. Verfahren zur Auswahl eines Getreides, umfassend das Bereitstellen eines Antikörpers nach Anspruch 3 und das Auswählen des Getreides basierend auf Reaktivität.

10. Pharmazeutische Zusammensetzung, umfassend ein Peptid nach Anspruch 1 oder Anspruch 2.

11. Pharmazeutische Zusammensetzung nach Anspruch 10 zur Herbeiführung von Toleranz.

12. Pharmazeutische Zusammensetzung nach Anspruch 10 zur Behandlung von Gluten-Empfindlichkeit.

13. Pharmazeutische Zusammensetzung nach Anspruch 10 zur Eliminierung Gluten-empfindlicher T-Zellen.

## Revendications

1. Peptide recombinant ou synthétique isolé comprenant la séquence d'aminoacides PFRPEQPYPQPQPQ.

2. Peptide recombinant ou synthétique isolé selon la revendication 1 où ledit peptide est flanqué par des aminoacides représentant des sites de maturation d'antigène.

3. Anticorps reconnaissant spécifiquement la séquence d'aminoacides PFRPEQPYPQPQPQ dans un peptide selon la revendication 1 ou la revendication 2.

4. Immunotest comprenant un anticorps selon la revendication 3.

5. Kit de diagnostic comprenant
- un anticorps selon la revendication 3 et
- un moyen de détection approprié.

6. Kit de diagnostic selon la revendication 5 pour détecter dans un aliment, des composants alimentaires ou des échantillons biologiques la présence d'un peptide dérivé de prolamine impliqué dans une entéropathie immunitaire liée aux aliments.

7. Kit de diagnostic selon la revendication 6 où ladite entéropathie immunitaire est choisie dans le groupe de la maladie coeliaque, la sprue tropicale, la giardiase et les allergies alimentaires de l'enfance.

8. Procédé pour réduire la quantité de peptides dérivés de prolamine toxiques dans un aliment ou des composants alimentaires comprenant l'incubation d'un anticorps selon la revendication 3 avec ledit aliment ou composant alimentaire.

9. Procédé pour sélectionner une céréale comprenant la fourniture d'un anticorps selon la revendication 3 et la sélection de ladite céréale sur la base de la réactivité.

10. Composition pharmaceutique comprenant un peptide selon la revendication 1 ou la revendication 2.

11. Composition pharmaceutique selon la revendication 10 pour l'induction d'une tolérance.

12. Composition pharmaceutique selon la revendication 10 pour le traitement de la sensibilité au gluten.

13. Composition pharmaceutique selon la revendication 10 pour l'élimination de cellules T sensibles au gluten.
